Europäisches Patentamt

European Patent Office

Office européen des brevets

(19)

Publication number: **0 308 900**

**A2**

# EUROPEAN PATENT APPLICATION

(21) Application number: 88115491.8

(22) Date of filing: 21.09.88

(51) Int. Cl.⁴: **A61B 19/02**

(30) Priority: 21.09.87 JP 143086/87
04.12.87 JP 305761/87
26.07.88 JP 184610/88

(43) Date of publication of application:
29.03.89 Bulletin 89/13

(84) Designated Contracting States:
AT DE FR GB IT

(71) Applicant: NIPPON PETROCHEMICALS CO.,
LTD.
3-1, Uchisaiwai-cho 1-chome Chiyoda-ku
Tokyo(JP)

(72) Inventor: Yamada, Hideaki
4-3-23, Yukinoshita
Kamakura-shi, Kanagawa-ken(JP)
Inventor: Kimura, Masato
23-203 Hanezawa Danchi, 921, Endo
Fujisawa-shi, Kanagawa-ken(JP)
Inventor: Oka, Masataka
2-6-6, Tsujido
Fujisawa-shi, Kanagawa-ken(JP)
Inventor: Saito, Kohta
8-11, Isogo, Isogo-ku
Yokohama-shi, Kanagawa-ken(JP)

(74) Representative: Schmidt-Evers, Jürgen,
Dipl.-Ing. et al
Patentanwälte Dipl.-Ing. H. Mitscherlich
Dipl.-Ing. K. Gunschmann Dipl.-Ing.
Dr.rer.nat. W. Körber Dipl.-Ing. J.
Schmidt-Evers Dipl.-Ing. W. Melzer Postfach
26 01 32
D-8000 München 26(DE)

(54) Disposable tray for medical use.

(57) A disposable tray (4) for medical use comprising at least two layers (1, 2, 3) formed of thermoplastic resin and being sterilized and when it is intended to be used, the first surface layer (1) thereof is peeled, and after use it is disposed by being folded into two. In use, liquid medicines are contained in recessed portions (6) made therein and medical instruments are placed to rest thereon, and filthy matters are received in a large recessed portion (7) of the tray (4).

FIG.1

## DISPOSABLE TRAY FOR MEDICAL USE

### BACKGROUND OF THE INVENTION

This invention relates to a disposable thermoplastic tray for medical use which requires neither cleaning operation nor sterilization treatment.

It has been usual hitherto that a tray used for keeping therein liquid medicines and medical instruments for a medical treatment is generally made of stainless steel or heat resisting synthetic resin, and is either sterilized by boiling immediately before its actual use or taken out from a safe-keeping shelf in which it has been kept in a sterilized condition, and then put to a medical use for placing medicine bottles and sterilized medical instruments thereon.

However, the conventional tray described above has not only such an inconvenience that it has to undergo sterilization by boiling, which is a troublesome and time-consuming process, each time it is used, or a special facility has to be provided for keeping it sterile for later use, but also such a sanitary problem that it is possible for saliva or blood of other patients to get accidentally mixed up in the liquid medicines. Furthermore, in view of the recently spreading rumor that one could contract AIDS (Acquired Immunodeficient Syndrome) when receiving a dental treatment or the like, it is very important to give a patient such an impression that medicine bottles and instruments used in a medical treatment are all perfectly clean so that the patient is relieved of any possible psychological fear of getting infected with such a disease during the treatment.

### OBJECT AND SUMMARY OF THE INVENTION

It is the object of the present invention to provide a disposable tray for medical use which is free from such disadvantages as observed with the conventional one.

To achieve the above object, the present invention provides a disposable tray for medical use which is characterized in that a tray body thereof comprises at least two layers formed of thermoplastic resin through a thermoforming process performed at a temperature of 150°C or higher, a surface layer thereof being able to be peeled from the next underlying layer, and has recessed portions to contain liquid medicines therein and an instrument resting section for resting medical instruments thereon, said instrument resting section being provided with ridges to support the medical instruments. In the above-described tray, there is also provided in the tray body another large recessed portion to keep filthy matters therein. As a means to be provided with the recessed portions, the tray body is formed to be rectangular and has a raised section formed at one end thereof in its longitudinal direction, in which section the recessed portions are provided. In order to make it convenient to carry the tray after use, the tray body is further provided with a folding groove so formed as to be perpendicular to said ridges and permit the tray to be folded into two while there is also provided at one end of the tray body a peel-off tab portion to facilitate peeling off the surface layer thereof. In addition, it is so formed that the bottom surfaces of the recessed portions and that of the instrument resting section may be located on the same plane so as to make the tray rest stably on a treatment table or the like. Preferably, the tray body is formed of three layers, of which the outermost layer is a layer compounded with at least one kind of additives or of inorganic fillers, the intermediate layer is a layer compounded with neither an additive nor an inorganic filler, and the innermost or surface layer is a layer also compounded with neither an additive nor an inorganic filler.. It is also preferable that the tray is characterized in that peel-off strength of the surface layer from the next underlying layer is in the range of 30 to 200g/20mm W. and that of the other layer portion thereof than said surface layer is in the range of 0.2 to 5.0 mm, and that thickness of the surface layer is in the range of 0.01 to 0.5mm, that the surface layer is different in color from the other layers so as to let one tell easily whether or not a particular tray has been used, and that, to manufacture the tray, the tray body is made to consist of a multi-layer sheet formed through a co-extrusion molding process or of a multi-layer injection molded product, the surface layer thereof being made of low-density polyethylene and most of other layer portions than the surface layer being made of polypropylene.

When the tray is to be used, the surface layer thereof is peeled off from the next underlying layer and the tray is then placed on a table or the like, and a necessary amount of each required liquid medicine is put in one of the recessed portions of the tray body whose new surface layer is now constituted by the former next underlying layer while sterilized instruments are placed in the instrument resting section thereof. Filthy matters such as used gauze are kept in the large recessed portion provided for that purpose.

If the tray is provided with a folding groove to permit folding the tray body into two along the direction perpendicular to the ridges, the tray body is folded into two along said folding groove when the treatment is over, and then hand-carried.

If the tray is provided with a peel-off tab portion at one end of the tray body, the surface layer is peeled off easily from the next underlying layer by holding the tab portion and pulling it.

When the surface layer of the tray body is colored differently from the other layer portion than said surface layer, the surface color is different between when the surface layer is peeled off and when it is yet to be peeled off, so that it can be easily distinguishable whether or not a particular tray has been already used.

BRIEF DESCRIPTION OF THE DRAWINGS

Fig. 1 is a general plan view of one embodiment of a tray for medical use according to this invention;

Fig. 2 is a sectional view taken along the line A-A thereof;

Fig. 3 is a sectional view taken along the line B-B thereof;

Fig. 4 is an enlarged sectional view taken along the line C-C thereof; and

Fig. 5 is a sectional view showing a condition in which the peel-off tab portion shown in Fig. 4 has been pulled up wards for peeling off the surface layer.

DETAILED DESCRIPTION OF PREFERRED EMBODIMENT

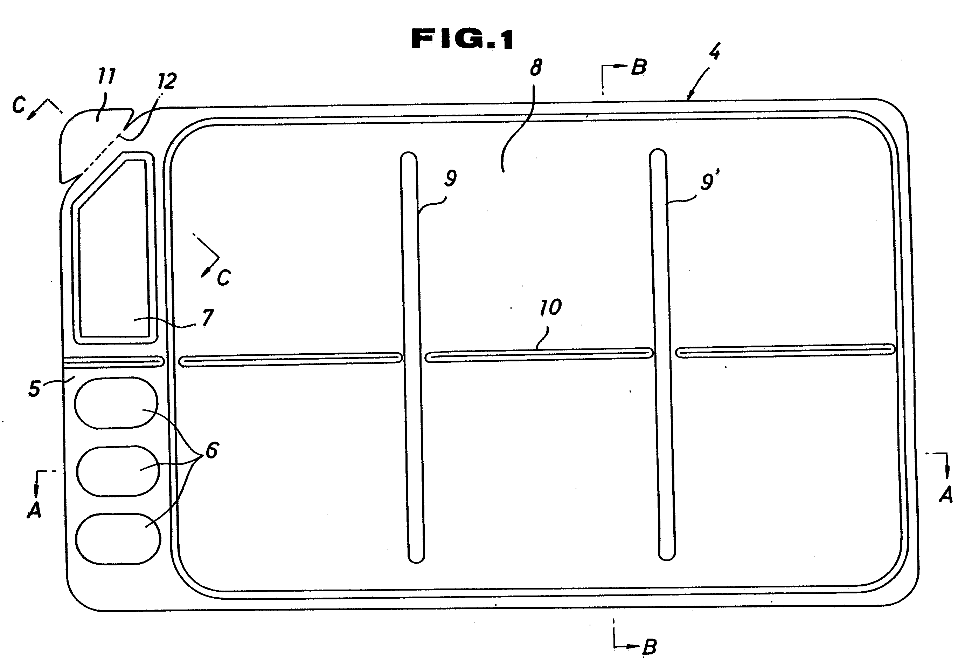

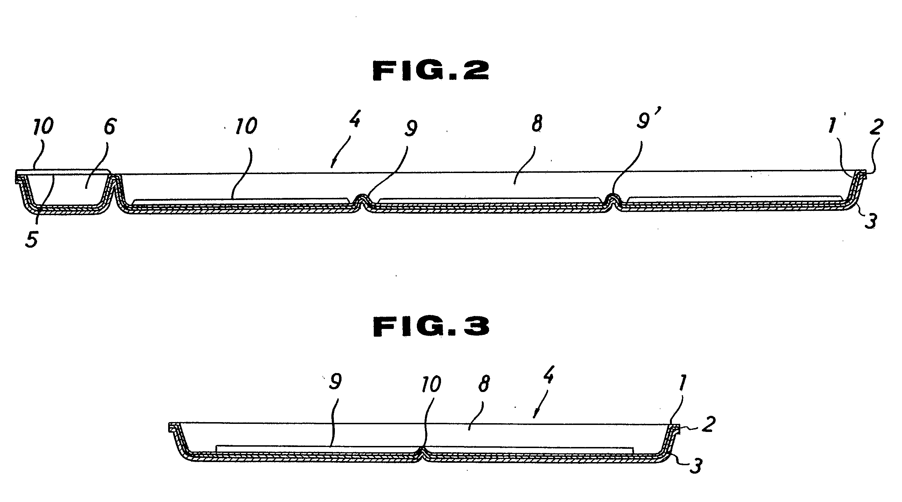

Now, embodying examples of the present invention will be explained more in detail with reference to the accompanying drawings. Fig. 1 is a general plan view of a tray according to this invention, Fig. 2 is a sectional view taken along the line A-A thereof, and Fig. 3 is another sectional view taken along the line B-B thereof. This particular example shows an embodiment of the present invention in which the tray comprises three layers, each of which is made of thermoplastic resin. A first layer (1) constituting the surface layer, that is, the innermost layer, ranges in thickness from 0.01 mm to 0.5 mm and is made of polyethylene, polypropylene, maleic anhydride modified polyethylene, maleic denatured polypropylene or the like while a second layer (2) and a third layer (3) which serve to hold the tray in its original shape ranges in thickness from 0.2 mm to 5.0 mm, for instance, and are made of the same kind of resin such as polypropylenen, polyethylene, polystyrene, polyvinyl chloride, polycarbonate, plyester, polyvinylidene - chloride, polyvinyl alochol, saponified ethylene vinylacetate copolymer product or polyamide. All these three layers (1), (2) and (3) are molded into a laminated structure by a co-extrusion process or the like which is carried out at a temperature (150° C or higher) required to get rid of bacteria (including viruses) and is then formed into the shape of a tray by a thermoforming process in order to produce a tray body (4). Of these three layers, the first layer (1) constituting the surface layer and the second layer (2) constituting the next underlying layer are formed respectively of a suitable combination of the thermoplastic resins selected from the above mentioned respective groups so that said peel-off strength may be in the range of 30 - 200g/20mm W. and the two layers can be peeled from each other when a peeling-off action is applied thereto from outside, although they look integrally constructed in appearance.

Of the second (2) and the third (3) layers serving to hold the tray in its original shape in this embodiment example, the third layer (3) includes at least one kind of such varied additives and compounding ingredients as an antioxidant, an ultraviolet absorbent and/or the like for improving durability of the tray, an organic or inorganic pigment or dye for coloring the same, and calcium carbonate, talc or the like for increasing rigidity thereof, while the second layer (2) includes no such additives or compounding ingredients as included in the third layer, thereby minimizing possible transfer of said additives or compounding ingredients therethrough. The first layer (1) constituting the original surface layer does not include such additives and compounding ingredients either so as to provide a perfectly clean surface of the tray body (4) after the first layer (1) is peeled off. If desired, the surface layer may be formed of a two-layer structure, the outer layer thereof including said additive or the like and the inner layer thereof including no additive or the like, so that a new surface of the tray appearing after the original surface or the first layer is peeled off may be kept substantially clean.

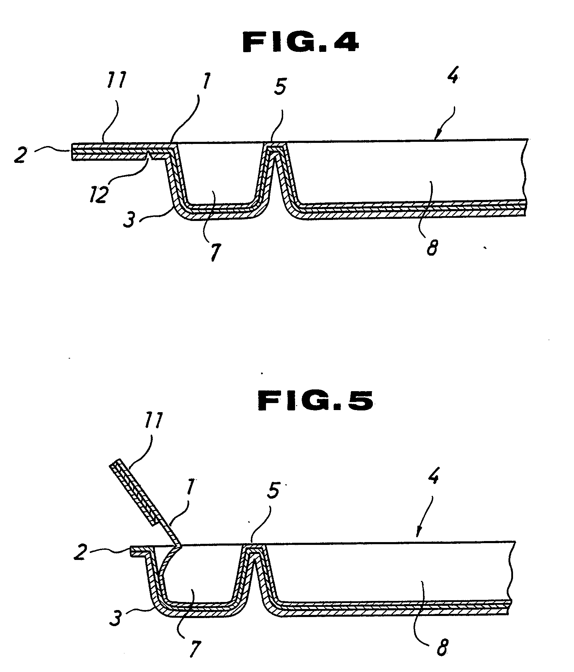

As described in the foregoing, the tray body (4) thermally formed in the shape of a tray is preferred to come in a rectangular form and further has a raised section (5) formed at one-end portion thereof in its longitudinal direction, said raised section (5) not exceeding in height the edge of the tray body and in the raised section there are provided recessed portions (6) for keeping liquid medicines and a compratively large recessed portion (7) for containing filthy matters. Other portions of the tray body than said raised section (5) are used as an instrument resting section (8). The bottom surfaces of this instrument resting section (8) and the recessed portion (7) are approximately on the same plane. The bottom of said instrument resting section (8) has at two locations thereon instrument-supporting ridges (9), (9) so disposed in parallel to each other as to extend in the direction perpendicular to the longitudinal direction of the tray body. In addition, the tray body (4) has a folding groove (10) so provided as to extend along the longitudinal center line thereof so that the tray body can be folded into two laterally along said groove. As shown in Fig. 4, a peel-off tab portion (11) is provided at one corner portion of the tray body (4) to facilitate peeling off the first layer constituting the surface layer, said tab portion being formed to have a cut groove (12) just deep enough to cut the third (3) and the second (2) layers all through but leaving the first layer (1) intact so that the tab (11) may be continued only to the first layer (1) constituting the surface layer and thus the first layer (1) can be easily peeled off as indicated in Fig. 5.

In the foregoing example, the first (1) and the third (3) layers are colored differently from each other, for instance, the first layer in blue and the third in white, so as to let one tell easily whether or not the tray has been already used.

When the tray is going to be used, the tab portion (11) is pulled to peel off the first layer (1) which is the original surface layer. With the first layer (1) so removed, there appears the second layer (2) whose surface is in a sterile condition since the molding thereof and thus requires no sterilization treatment so that the tray can be immediately placed on a treatment table or the like to be readily put to a medical use in which it holds required liquid medicines in its recessed portions (6) and rests necessary medical instruments on the ridges (9), (9) in the instrument resting section (8) thereof. The used gauze or the like is retained in the recessed portion (7).

Next, after the medical treatment is finished, the tray body (4) is folded into two as if to wrap up therein the used medical instruments and, together with them, hand-carried to the place where the used instruments are sterilized. After the instruments are removed from the tray body at that place, the tray now emptied of the medical instruments is disposed of.

In the foregoing embodiment example, the tray body is formed in the shape of a rectangle but it goes without saying that the tray can be formed into any other suitable shape such as a circular or a semicircular one.

More concrete examples of manufacturing a disposable tray for medical use according to the present invention are described as follows:

Example 1:

Polypropylene having melt flow rate (MFR) of 4.0g/10 min. and density (d) of 0.91g/cm³ and known under the trade name of "NISSEKI POLYPROPYLENE J 130" (manufactured by Nippon Petrochemicals Co., Ltd.) was used as the thermoplastic resin for forming the second (2) and the third (3) layers. The resin for the third layer was compounded with 0.3 Wt.% of an antistatic agent and 0.5 Wt.% of titanium white while the resin for the second layer (2) was compounded with neither of said additives. Low-density polyethylene having MFR of 1.0g/10 min. and density of 0.92g/cm³ and known under the trade name of "NISSEKI REXLON F 22" (manufactured by Nippon Petrochemical Co., Ltd.) was used as the thermoplastic resin for forming the first or surface layer (1) and was compounded with 0.2 Wt.% of a blue organic pigment. The laminar flows of the materials were joined together and melted in the die at the resin temperature of 240°C and formed into a three-layer sheet by a co-extrusion process using a three-layer T-die, chill roll method.

As for the size of the sheet, the third layer (3) was 0.1 mm thick, the second layer (2) was 0.3 mm thick and the first or surface layer (1) was 0.1 mm thick.

Using a contact heating and vacuum forming process, the sheet obtained through the foregoing co-extrusion molding process was then formed in vacuum condition at the preheating temperature of 160°C into the shape of a tray measuring 250 mm in length, 140 mm in width and 11 mm in depth in such a way that the surface layer (1) should constitute the inner wall of the tray, and then was cut off along the outer periphery of said tray with a cut-off edge, thereby obtaining the tray body (4) having the tab portion (11). Concurrently with the above cut-off operation, the third (3) and the second (2) layers were cut by a lower cutting edge to provide the tab portion (11) with the cut groove (12).

Using the above-described tray body (4), a test piece was prepared therefrom and tested for evaluating its peel-off strength and sterile condition. The peel-off strength was found to be 105g/20mm W. and when the tab portion (11) was bent backwards toward the inner side of the tray body, only the surface layer (1) covering the surface of the second layer of the tray body (4) was easily peeled off completely from the surface of the second layer, said peel starting from one end of the first layer and causing no deformation to the tray body (4). As for the sterile condition thereof it was evaluated by using the thioglycollate culture medium for a sterile test prescribed under an aseptic test method of the Japanese Pharmacopoeia in the following manner: After the surface layer (1) was peeled off, the thioglycollate culture medium was applied onto the tray body (4). The tray body was then left to remain for 7 days in an aseptic box constantly maintained at 31°C to observe visually whether or not bacteria would grow thereon. As a result of this observation, it has been confirmed that the tray body (4) was kept clean, because no growth of bacteria was recognized in this test.

Example 2:

For molding the second (2) and the third (3) layers, polystyrene having MFR of 2.7g/10 min. and known under the trade name of "STYRON 475D" (manufactured by Asahi Chemical Industry Co., Ltd.) was used as the thermoplastic resin to form the second (2) and the third (3) layers and was compounded with 0.3 wt% of an antistatic agent such as a polyoxyethylene alkyl amine (as sold under the trade name Electrostripper TS-5 manufactured by Kabushiki Kaisha KAO). For molding the surface layer (1), maleic anhydride modified polyethylene having MFR of 0.8g/10 min. and density of $0.91g/cm^3$ and known under the trade name of "NISSEKI N POLYMER L 6033" (manufactured by Nippon Petrochemicals Co., Ltd.) was used as the thermoplastic resin to form said layer and was compounded with 0.2 wt% of a blue organic pigment such as phthalocyanine blue, known as Pigment blue 15 (C. I. No. 74160), as sold under the trade name Heliogen Blue K-7080, manufactured by BASF. The laminar flows of these materials were joined together and melted in the die at the resin temperature of 200°C and formed into a multi-layer sheet by a co-extrusion process using a two-layer T-die, chill roll method.

As for the size of the sheet, the second (2) and the third (3) layers were both 0.4 mm thick and the surface layer was 0.1 mm thick.

Using the foregoing vacuum forming process, the sheet obtained through the foregoing co-extrusion process was then thermally formed in vacuum condition at the preheating temperature of 150°C into the shape of a tray in such a way that the surface layer (1) of the sheet should constitute the inner wall of the tray and then was cut off along the outer periphery of said tray with the cut-off edge, as in the Example 1, thereby obtaining the tray body (4) with the tab portion (11).

Concurrently with the above cut-off operation, the third (3) and the second (2) layers were cut by the lower cutting edge to provide the tab portion (11) with the cut groove (12).

Using the above-described tray body (4), a test piece was prepared therefrom and tested to evaluate the peel-off strength and sterile condition thereof. The peel-off strength was found to be 160g/20mm W. and when the tab portion (11) was bent backwards toward the inner side of the tray body, only the surface layer (1) covering the surface of the second layer of the tray body (4) was easily peeled off, said peel starting from one end of the layer and causing no deformation to the tray body (4). The sterile condition was checked in the same manner as in the Example 1 to find out that the new surface of the tray body appearing after the peel-off was kept clean.

Example 3:

For molding the second (2) and the third (3) layers, polyvinyl chloride known under the trade name of "SHIN-ETSU PVC" (manufactured by Shin-Etsu Chemical Co., Ltd.) was used as the thermoplastic resin and was compounded with 0.3 Wt.% of an antistatic agent. For molding the surface layer (1), maleic denatured polyethylene having MFR of 0.8g/10 min. and density of $0.91g/cm^3$ and known under the trade name of "NISSEKI N POLYMER L 6033" (manufactured by Nippon Petrochemicals Co., Ltd.) was used as the thermoplastic resin and was compounded with 0.2 Wt.% of a blue organic pigment. The laminar flows of these materials were joined together and melted in the die at the resin temperature of 190°C and formed into a multi-layer sheet by a co-extrusion process using two-layer T-die, chill roll method.

As to the size of the sheet, the second (2) and the third (3) layers were 0.4 mm thick respectively and the surface layer (1) was 0.1 mm thick.

Using the foregoing vacuum forming process, the sheet obtained through the foregoing co-extrusion process was then thermally formed in vacuum condition at the preheating temperature of 140°C into the shape of a tray in such a way that the surface layer (1) of the sheet should constitute the inner wall of the tray, and then cut off along the outer periphery of said tray with the cut-off edge, as in the Example 1, thereby obtaining the tray body (4) with the tab portion (11).

Concurrently with the above cut-off operation, the third (3) and the second (2) layers were cut by the lower cutting edge to provide the tab portion (11) with the cut groove (12).

Using the above-described tray body (4), a test piece was prepared therefrom and tested to evaluate its peel-off strength and sterile condition. The peel-off strength was found to be 180g/20mm W. and when the tab portion (11) was bent backwards toward the inner side of the tray body, only the surface layer (1) covering the surface of the second layer of the tray body (4) was easily peeled off, said peel starting from one end of the layer and causing no deformation to the tray body (4). The sterile condition was checked in the same manner as in the Example 1 to find out that the new surface of the tray body (4) appearing after the peel-off was kept clean.

As described in the foregoing, a disposable tray for medical use according to this invention has such a construction that it can be readily put to a medical use without undergoing any sterilization treatment. This eliminates a troublesome sterilization treatment applied to a conventional tray of the kind each time it is to be used and additionally nunecessitates a special facility to keep the tray in the sterile condition, thus bringing about an economical advantage.

Another advantage thereof is that a patient can see the perfect cleanness of the tray for himself or herself and is completely relieved of a much talked-about fear of contracting AIDS while receiving a dental or surgical treatment since the tray gets its surface layer peeled off under the eyes of the patient when it is to be used, thus giving a strong impression of cleanliness, while the liquid medicines are used for each patient strictly on an individual basis to ensure the best sanitation.

Additionally, according to this invention, the tray has a folding groove provided to extend in the direction perpendicular to the ridges thereof so that the tray body can be folded into two after use to make it easy and convenient to hand-carry the used instruments therein. A peel-off tab portion is also provided at one end of the tray body in order to facilitate peeling off the first layer which constitutes the original surface layer. The bottom surfaces of the recessed portions and that of the instrument resting section are so formed as to be on the same plane, thus enabling the tray to rest stably on a treatment table or the like. As the surface layer of the tray body and at least one layer of the remainder portion thereof other than said surface layer are colored differently from each other, it is easy to tell whether or not a particular tray has been used, so that there is no danger of erroneously using the same tray again, this being one advantage of the present invention.

## Claims

1. A disposable tray for medical use characterized in that a tray body (4) comprises at least two layers formed of thermoplastic resin through a thermoforming process performed at a temperature of 150° C or higher, a surface layer (1) thereof being able to be peeled from the next underlying layer (2), and has recessed portions (6) for containing liquid medicines therein and an instrument resting section (8) for resting medical instruments thereon, said instrument resting section (8) being provided with ridges (9) for supporting said medical instruments.

2. A disposable tray for medical use according to claim 1, wherein the tray body (4) is further provided with another recessed portion (7) to keep filthy matters therein.

3. A disposable tray for medical use according to any one of claims 1 and 2, wherein the tray body (4) is formed in the shape of a rectangle and has a raised section (5) formed in one-end portion thereof, in which section the recessed portions (6) are provided.

4. A disposable tray for medical use according to any one of claims 1, 2 and 3, wherein a folding groove (10) along which the tray body (4) can be folded into two is provided to extend in the direction perpendicular to the ridges (9).

5. A disposable tray for medical use according to any one of claims 1, 2, 3 and 4, wherein a peel-off tab portion (11) is provided at one end of the tray body (4) so as to facilitate peeling off the surface layer (1).

6. A disposable tray for medical use according to any one of claims 1, 2, 3, 4 and 5, wherein the bottom surfaces of the recessed portions (6) and that of the instrument resting section (8) are so formed as to be on the same plane.

7. A disposable tray for medical use according to any one of claims 1 through 6, wherein the tray body (4) comprises three layers (1, 2, 3), the outermost one (3) thereof being compounded with at least one kind of additives or of inorganic fillers and the intermediate one (2) thereof being compounded with neither an additive nor an inorganic filler.

8. A disposable tray for medical use according to any one of claims 1 through 7, wherein the surface layer (1) of the tray body (4) is a layer compounded with neither an additive nor an inorganic filler.

9. A disposable tray for medical use according to any one of claims 1 through 8, wherein the peel-off strength between the surface layer (1) and the next underlying layer (2) of the tray body (4) is 30 to 200g/20mm W.

10. A disposable tray for medical use according to any one of claims 1 through 9, wherein the thickness of the surface layer (1) of the tray body (4) is in the range of 0.01 to 0.5 mm and that of the layer portion (2, 3) other than said surface layer (1) is in the range of 0.2 to 5.0 mm.

11. A disposable tray for medical use according to any one of claims 1 through 10, wherein the surface layer (1) of the tray body (4) and the layer portion (2, 3) other than said surface layer (1) is different in color from each other.

12. A disposable tray for medical use according to any one of claims 1 through 11, wherein the tray body (4) is formed of a coextrusion molded multi-layer sheet or a multi-layer injection molded product.

13. A disposable tray for medical use according to any one of claims 1 through 12, wherein the surface layer (1) is formed of low-density polyethylene and the layer portion (2, 3) other than said surface layer (1) is formed of polypropylene.

**FIG.1**

EP 0 308 900 A2

EP 0 308 900 A2

FIG.3

FIG.2

# FIG.4

# FIG.5